# EUROPEAN PATENT SPECIFICATION

(11) **EP 2 725 667 B1**
(45) Date of publication and mention of the grant of the patent: **20.04.2016**
(21) Application number: 13187318.4
(22) Date of filing: 04.10.2013
(51) Int. Cl.: H01S 3/106, H01S 3/16, H01S 3/08, H01S 3/11

(54) **Multiple wavelength laser apparatus and control method thereof**
Laseranordnung mit mehreren Wellenlängen und Verfahren zur Steuerung dessen Laser
Laser à longueurs d'ondes multiples et sa méthode de contrôle

(30) Priority: 23.10.2012 JP 2012233752
(43) Date of publication of application: 30.04.2014
(73) Proprietor: Canon Kabushiki Kaisha, Tokyo 146-8501 (JP)
(72) Inventor: Ichihara, Shigeru, Tokyo, Tokyo 146-8501 (JP)
(74) Representative: TBK

(56) References cited:
- WO-A1-2010/005109
- WO-A1-2012/140864
- WO-A1-2013/018643
- WO-A1-2013/129108
- WO-A1-2013/132977
- CN-A- 101 872 934
- US-B1- 6 193 711
- UCHIDA T ET AL: "Analysis on the compensating thermal lensing effect using an convex mirror in vertical-cavity surface emitting lasers", JAPANESE JOURNAL OF APPLIED PHYSICS, JAPAN SOCIETY OF APPLIED PHYSICS, JP, vol. 43, no. 8B, 1 January 2004 (2004-01-01), pages 5933-5936, XP003010584, ISSN: 0021-4922, DOI: 10.1143/JJAP.43.5933
- SRIRANG MANOHAR ET AL: "<title>Region-of-interest breast images with the Twente Photoacoustic Mammoscope (PAM)</title>", PROCEEDINGS OF SPIE, vol. 6437, 7 February 2007 (2007-02-07), pages 643702-643702-9, XP055107398, ISSN: 0277-786X, DOI: 10.1117/12.699995
- SRIRANG MANOHAR ET AL: "<title>Speed-of-sound imaging in a photoacoustic imager</title>", PROCEEDINGS OF SPIE, vol. 6437, 7 February 2007 (2007-02-07), pages 64370R-64370R-8, XP055107399, ISSN: 0277-786X, DOI: 10.1117/12.700078

## Description

### BACKGROUND OF THE INVENTION

### Field of the Invention

The present invention relates to a laser apparatus and a control method thereof.

### Description of the Related Art

Photoacoustic tomography apparatuses (hereinafter, "PAT apparatus") for medical applications that use a tunable pulsed laser have been developed (see S. Manohar et al, Proc. of SPIE vol. 6437643702-1). A PAT apparatus is a measurement device that emits nanosecond laser pulses to a segment being measured, receives signals converted from ultrasound waves (photoacoustic waves) generated from the segment and analyzes the signals to obtain an image. PAT apparatuses are expected to enable in-vivo determination of presence or absence of a tumor through observation of congregating blood vessels, or in-vivo functional analysis through spectral measurement based on absorption coefficients of biological tissues in accordance with the wavelengths of emitted laser light.

Tunable lasers that use titanium sapphire crystal or alexandrite crystal are considered suitable as a light source of a PAT apparatus. Alexandrite lasers, in particular, can be directly excited by a flash lamp to achieve high energy output relatively easily due to the long fluorescence lifetime of the alexandrite crystal used as the laser medium.

On the other hand, the emission spectrum of a flash lamp is wide, ranging from ultraviolet to infrared, so that the consistency with the absorption spectrum of the laser medium is poor. Thermal energy of the flash lamp causes temperature changes in the laser medium, which leads to thermal lensing effect, wherein the light beam passing through the laser medium is refracted. Alexandrite lasers are known for significant thermal lensing effect as pointed out in a report relating to an alexandrite laser for a multi-wavelength resonance scattering lidar system used for observation of the atmosphere. Therefore, to achieve stable pulse emission, the thermal lensing effect needs to be reduced or stabilized.

The temperature distribution of the laser medium depends on the thermal energy given by the flash lamp and the cooling effect by circulating water around the laser medium. The laser medium is immersed in circulating water of a constant temperature, while the lamp energy is maintained constant to keep the thermal lensing power in a steady state. A method has been disclosed in US patent US-6,193,711 wherein, when changing the lamp energy, the average lamp energy is made constant per a plurality of pulses to achieve stable pulse emission.

With a PAT apparatus, by using laser light of two or more wavelengths, oxygen saturation of blood can be determined from a spectral difference between oxyhemoglobin and deoxyhemoglobin. When obtaining precise in-vivo functional information such as oxygen saturation using photoacoustic signals based on two wavelengths, it is preferable to keep the laser energy output of both wavelengths at the same level.
Patent Literature 1: U.S. Patent US-6,193,711
Non Patent Literature: S. Manohar et al., Proc. of SPIE vol. 6437 643702-1
Prior art which is related to this field of technology can be found e.g. in document WO 2012/140864 A1 disclosing laser apparatus and photoacoustic apparatus, in document US 6,193,711 B1 disclosing rapid pulsed Er:YAG laser, in document UCHIDA T. ET AL.: "Analysis on the compensating thermal lensing effect using a convex mirror in vertical-cavity surface-emitting lasers", JAPANESE JOURNAL OF APPLIED PHYSICS, JAPAN SOCIETY OF APPLIED PHYSICS, vol. 43, no. 8B, 1 January 2004, pages 5933-5936, XP 003010584, and in document WO 2010/005109 A1 disclosing photoacoustic measurement apparatus.

### SUMMARY OF THE INVENTION

The oscillation band of an alexandrite laser is from 720 nm to 800 nm, which matches the absorption band of oxyhemoglobin or deoxyhemoglobin, so that it is suited to measure oxygen saturation. On the other hand, as the gain intensity is largely different for each wavelength, the energy output level of the laser is largely different at around 750 nm and around 800 nm. In order to achieve stable pulse emission at the same level of output irrespective of the wavelength, therefore, the energy output of the flash lamp need to be controlled at the same time with the changing of the wavelengths.

U.S. Patent US- 6,193,711 discloses an effective method of in-vivo irradiation with Er:YAG laser pulses. An Er:YAG laser emits light at a wavelength of 2940 nm, which is highly reactive to water in biological tissue and generates little heat, and therefore is suitably used for laser treatment. According to the method of achieving stable pulse emission disclosed in U.S. Patent US-6,193,711, the average energy output of the flash lamp for the pulses included in a pulse train is made constant, so as to make the average thermal energy given by the flash lamp to the Er:YAG laser medium constant.

However, unlike lasers using titanium sapphire or alexandrite with a broad gain band, the Er:YAG laser emits light at a fixed wavelength. U.S. Patent US-6,193,711 relates to a method of controlling a laser of a fixed wavelength. The configuration shown in U.S. Patent US-6,193,711 does not allow the wavelength to be readily changed and therefore cannot be used for in-vivo functional diagnosis by PAT that uses a difference in the absorption characteristics that biological tissue exhibits to different wavelengths.

In spectral measurement using PAT, on the other hand, a method of driving a laser that allows more accurate in-vivo measurement from the start of irradiation is required.

The present invention was made in view of the problem described above. The present invention is developed to achieve stable pulse output by a tunable pulsed laser irrespective of the wavelength.

The present invention in its first aspect provides a laser apparatus as specified in the respective claims.

The present invention in its second aspect provides a control method for laser apparatus as specified in the respective claims.

According to the present invention, a tunable pulsed laser can output stable pulses irrespective of the wavelength.

Further features of the present invention will become apparent from the following description of exemplary embodiments with reference to the attached drawings.

### BRIEF DESCRIPTION OF THE DRAWINGS

FIG. 1 is a configuration diagram illustrating one embodiment of a laser apparatus of the present invention;
FIG. 2A and FIG. 2B show a pulse pattern in one embodiment of the laser drive method of the present invention;
FIG. 3 is a configuration diagram illustrating one embodiment of a laser apparatus of the present invention;
FIG. 4 is a configuration diagram illustrating one embodiment of a laser apparatus of the present invention;
FIG. 5A and FIG. 5B show a comparative example of a pulse pattern;
FIG. 6 shows a comparative example of a pulse pattern;
FIG. 7 shows a pulse pattern in one embodiment of the laser drive method of the present invention;
FIG. 8 shows one embodiment of the laser drive method of the present invention;
FIG. 9 is a configuration diagram illustrating one embodiment of a laser apparatus of the present invention used for photoacoustic measurement; and
FIG. 10 is a configuration diagram illustrating one embodiment of a PAT apparatus.

### DESCRIPTION OF THE EMBODIMENTS

Preferred embodiments of the present invention will be hereinafter described with reference to the drawings. Note, it is not intended to limit the scope of this invention to the specifics given below, and the sizes, materials, shapes, and relative arrangements or the like of constituent components described below should be changed as required in accordance with the configurations and various conditions of the apparatus to which the invention is applied.

The present invention can be understood as a tunable laser apparatus, and a control method of the same. The present invention can be applied to a photoacoustic apparatus (PAT apparatus) including such a tunable laser apparatus as one constituent element. Namely, it is a PAT apparatus that has a tunable laser apparatus according to the present invention to irradiate an object with laser light to obtain a photoacoustic wave that is generated from a light absorbing part inside the object by the photoacoustic effect and propagates through the object. With the use of the tunable laser apparatus according to the present invention, the wavelength of light emitted to the object can be changed simply in a stable manner, so that presence of various tissues (light absorbing parts) having different light absorption characteristics can be identified as property information. The obtained information can be displayed to be used for diagnosis.

The acoustic waves referred to in the present invention are typically ultrasound waves, including elastic waves that are called sound waves, ultrasound waves, or acoustic waves. An acoustic wave generated by the photoacoustic effect is referred to as a "photoacoustic wave", or a "light-induced ultrasound wave".

### (Configuration of apparatus)

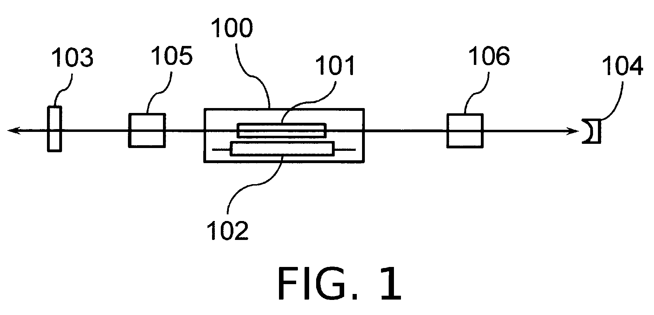

FIG. 1 shows one example of the configuration of the tunable laser used in the present invention. A cavity is formed by an output mirror 103 and a reflective mirror 104. An excitation chamber 100, a Q-switch 105, and a wavelength selector 106 are arranged inside the cavity. A rod-shaped laser medium 101 and a flash lamp 102 (excitation lamp) for exciting the laser medium 101 are arranged adjacent each other in the excitation chamber 100. Part of the emission energy from the flash lamp 102 is absorbed by the laser medium 101 to produce an inverted distribution, and the loss in the cavity is decreased by the Q-switch 105 at a desired timing to emit a pulse of light. The laser medium 101 is a laser crystal having a high gain bandwidth, and the wavelength selector 106 allows selection of a wavelength of output pulses.

Examples of applicable tunable pulsed lasers excited by a flash lamp are titanium sapphire lasers and alexandrite lasers. These tunable pulsed lasers have an oscillation wavelength band that matches the frequency bands corresponding to the absorption characteristics of oxyhemoglobin and deoxyhemoglobin, so that they can favorably be used to detect oxygen saturation of blood. Alexandrite lasers, in particular, can readily be excited by a flash lamp and output high energy per pulse. In the following example, the laser medium will be described as alexandrite, but the present invention is not limited to alexandrite lasers.

### (Thermal lensing effect)

In lasers that have a flash lamp as the excitation source, it is essential to make the thermal lensing power constant to achieve stable pulse emission. Thermal lensing will be described in detail below. When light emitted from the flash lamp is absorbed by the laser medium, heat is generated and changes the temperature of the laser medium. The densities and refractive indexes of portions where light was absorbed and the surrounding portions change in accordance with the temperature distribution at this time. The temperature of the laser medium depends on the temperature of the water circulating around the laser medium and the thermal energy emitted from the flash lamp. Generally, the temperature of the laser medium differs between a central part and peripheral parts so that the refractive index differs largely between the center and periphery, because of which the medium exhibits a lens effect. This phenomenon in the laser medium where a thermal lens is created is called thermal lensing effect.

### (Example of control of maintaining a steady temperature of the laser medium )

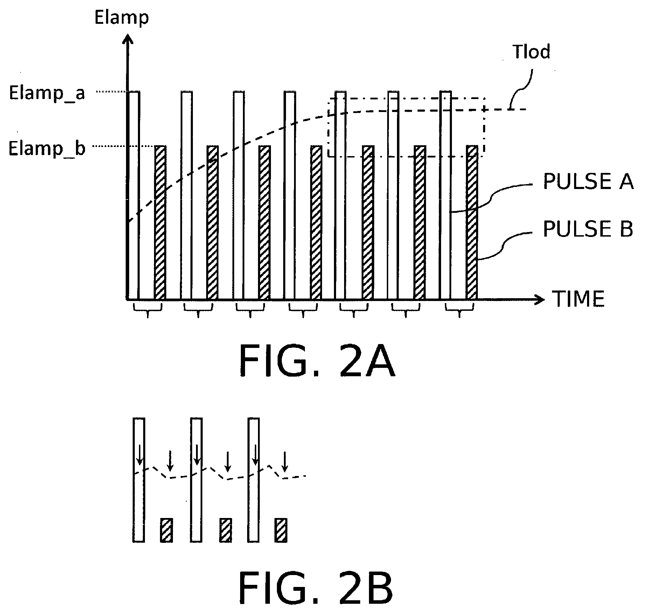

One embodiment of the laser drive method of the present invention will be described below. An example of a pulse pattern of light emitted at two different wavelengths with about the same energy output will be roughly described with reference to FIG. 2A. In FIG. 2A, the horizontal axis represents time (T), while the vertical axis represents emission energy (Elamp) of the flash lamp 102.

In the following description, pulses of laser light emitted with an emission energy of Elamp_a at wavelength λa will be referred to as "pulse A", while pulses of laser light emitted with an emission energy of Elamp_b at wavelength λb will be referred to as "pulse B". The object is irradiated repeatedly with a pulse train consisting of combinations of pulses A and pulses B. The energy levels Elamp_a and Elamp_b are determined such that the energy output of pulses emitted at different wavelengths will be substantially the same. Wavelength λa and wavelength λb can be considered as a first wavelength and a second wavelength in the definition of the present invention.

The broken line in FIG. 2A schematically represents the temperature change of the laser medium (rod). Since there is a temperature distribution inside the laser medium, the temperature on the center axis at an end face of the medium (Tlod) is used here as the representative temperature of the laser medium. The laser medium 101 is immersed in circulating water that fills the excitation chamber 100. The temperature of the circulating water is kept constant before the flash lamp is turned on.

When the flash lamp 102 is turned on, the temperatures of the laser medium and the circulating water rise. Pulses A and pulses B are emitted with respective emission energies of Elamp_a and Elamp_b set such that the energy output of the pulses at wavelengths λa and λb will be the same. As the pulse train of pulses A and pulses B is repeated, the laser medium temperature Tlod gradually rises to a steady state.

FIG. 2B shows an upper right part encircled by a one-dot chain line of FIG. 2A to a larger scale. The arrows indicate the timing of the Q-switch 105 pulse emission. Since the pulses A and pulses B are emitted with different emission energies of the lamp, the laser medium temperature Tlod changes slightly after each emission. However, as the average lamp energy is constant throughout the pulse train, the variation of Tlod is very small. Accordingly, the thermal lensing power of the laser medium is substantially constant, and both pulses A and pulses B are emitted stably. When the laser medium temperature is in a steady state and always the same pulse train is repeated, the laser medium temperature is substantially constant throughout each of pulses A and pulses B. Therefore, there is little difference in the light characteristics between pulses having the same wavelength.

While the temperature change of the laser medium is largely affected by the emission from the flash lamp, not just the emission intensity but the emission interval is also a significant temperature control factor. In the case of pulse lasers, the higher the repetition frequency, the shorter the emission interval of the flash lamp in a pulsed laser, so that the laser medium is steadily subjected to thermal energy and thus its temperature is largely affected. More specifically, the higher the repetition frequency, the smaller the effect of thermal relaxation of the laser medium, so that the laser medium temperature is stabilized.

A preferable range of repetition frequency is discussed below. From the point of view of stabilizing the laser medium temperature, a repetition frequency of 10 Hz or more, preferably 20 Hz or more, should be used, although it depends on the size of the laser medium and thermal properties such as specific heat of the medium. On the other hand from the point of view of designing a flash lamp-excited laser apparatus, although it depends on the voltage applied to the lamp, a repetition frequency of 100 Hz or less is commonly used in view of the power source capacity and the like.

Therefore, in an application as a PAT apparatus, the repetition frequency should preferably be 10 Hz to 50 Hz for the laser apparatus of the present invention, so as to avoid large load on the apparatus as well as to achieve stable pulse output.

### (Compensation for the thermal lensing effect)

While the laser medium temperature can be brought to a steady state by the laser drive method shown in FIG. 2A and FIG. 2B, the medium is not at thermal equilibrium, because the light from the flash lamp is emitted in pulses. A temperature change of more than a certain amount may cause variation of thermal lensing power that represents the degree of the thermal lensing effect.

However, such variation of thermal lensing power can be minimized to a very small range by reducing temperature changes of the laser medium as much as possible. If the variation of thermal lensing power is very small, it is possible to compensate for the thermal lensing effect by providing a certain curvature to the reflective mirror. Namely, stable pulse emission is ensured by configuring the cavity to be a stable resonator, which is determined by the curvatures of the output mirror 103 and the reflective mirror 104.

More specifically, the reflective mirror 104 is given a curvature as shown in FIG. 1, for example, to compensate for the thermal lensing effect. In this case, it is necessary for the light beam to enter the center of the reflective mirror 104 perpendicularly. The wavelength selector 106 should preferably be of the type that uses a birefringent filter formed by parallel flat plates and rotated in its plane, as the light beam is unlikely to be misaligned in such a system. A wavelength filter that allows a desired wavelength to transmit can also favorably be used as the wavelength selector 106.

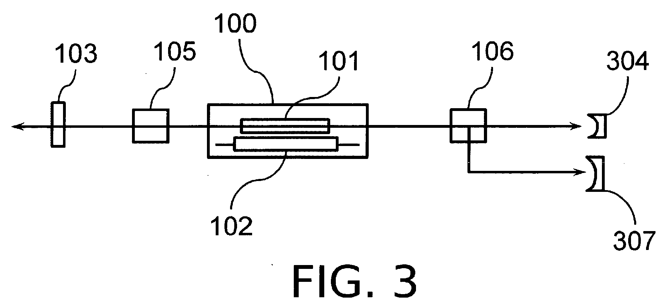

If the repetition frequency is less than 10 Hz, the laser medium temperature is more prone to change by thermal relaxation, although it depends on the difference in the emission energy between pulses A and pulses B. If there is a large difference in the laser medium temperature between pulses A and pulses B, then a laser resonator design shown in FIG. 3 should preferably be used for compensating for the thermal lensing effect suitably for respective pulses.

### (Examples of wavelength selectors)

In FIG. 3, the rod-shaped laser medium 101 and the flash lamp 102 for exciting the laser medium 101 are arranged adjacent each other in the excitation chamber 100. As shown in the drawing, the wavelength selector 106 branches the optical path so that a cavity is formed by the output mirror 103 and a reflective mirror 304, and another cavity is formed by the output mirror 103 and a reflective mirror 307. Inside the cavity are arranged the excitation chamber 100 and the Q-switch 105 along the common optical path from the output mirror 103 to the wavelength selector 106. By this branching of the optical path, light for pulses A is reflected by the reflective mirror 304, while light for pulses B is reflected by the reflective mirror 307. The two reflective mirrors each have a curvature in accordance with the thermal lensing power at respective wavelengths, to compensate for the thermal lensing effect for the pulses of respective wavelengths. The wavelength selector 106 may have any configuration as long as pulses are emitted stably and a desired wavelength can be selected from a plurality of options.

According to one known method of selecting a wavelength in which the optical path is branched, the wavelength selector 106 may include a fixed prism to use wavelength separation based on refractive index dispersion, and a shutter mechanism for selecting a light beam to be emitted. According to another method of selecting a wavelength, the wavelength selector 106 may use a parallel plate to branch the optical path and to select an optical path for the light beam to pass through. A dielectric film is provided on the reflective mirror for reflecting a desired wavelength.

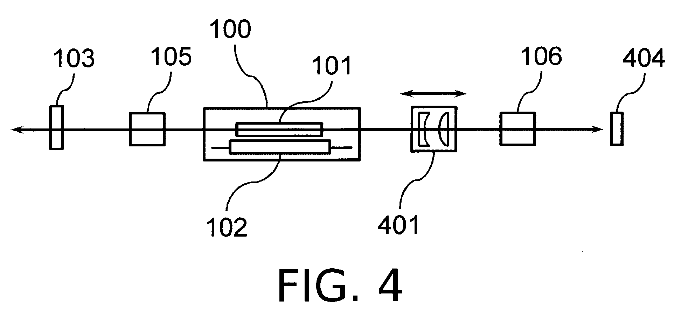

The thermal lensing power can also be compensated for by disposing a telescope 401 between the wavelength selector 106 and the excitation chamber 100 as shown in FIG. 4 and adjusting the distance between the lenses in the telescope 401. In this case, a reflective mirror 404 having a flat surface can be used. With the use of such a mirror 404 with a flat surface as the reflective mirror, there is no need to direct the light beam to the center of the reflective mirror, so that the wavelength selector 106 can use a prism or the like for utilizing refractive index dispersion that involves changes of optical paths.

### (Another example of control of maintaining a steady temperature of the laser medium)

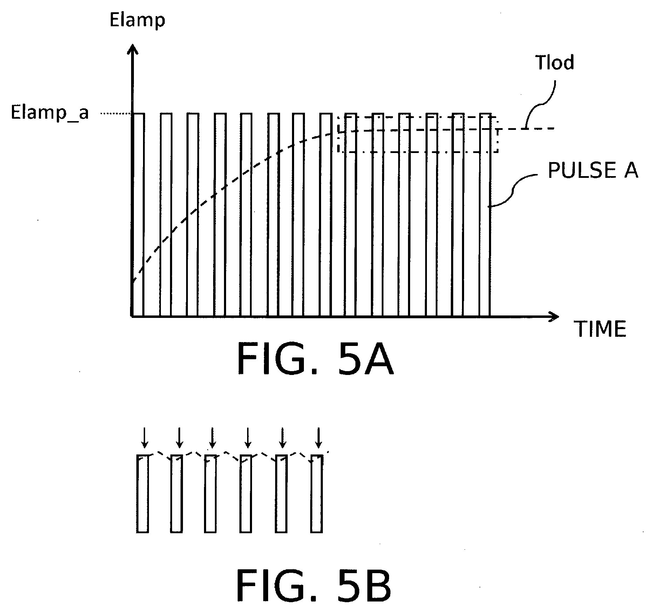

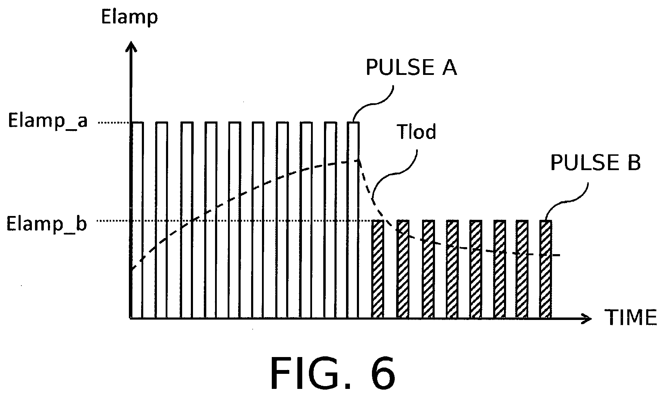

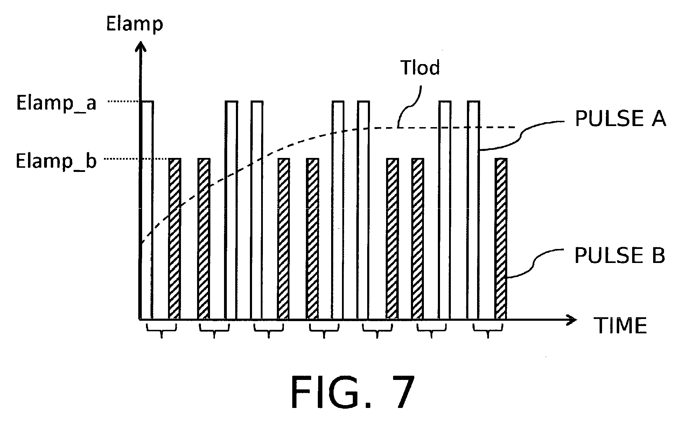

A pulse pattern different from the one shown in FIG. 2A and FIG. 2B will be described below. FIG. 5A, FIG. 5B and FIG. 6 show comparative examples, while FIG. 7 show a pulse pattern according to the present invention. The horizontal axis represents time (T), the vertical axis represents emission energy of the lamp (Elamp), and broken lines represent changes in the laser medium temperature (Tlod) in respective drawings.

FIG. 5A shows Comparative Example 1 of a pulse pattern. As pulses of light are emitted at a constant wavelength of λa and with a constant emission energy of Elamp_a, the laser medium temperature Tlod rises gradually to a thermally stable state.

FIG. 5B shows a part encircled by a one-dot chain line in FIG. 5A to a larger scale. The arrows indicate the timing of Q-switching. Since the flash lamp emits light in pulses, there are slight changes in the laser medium temperature. In the steady state, however, the temperature of the laser medium stays substantially constant during pulse emission, whereby the thermal lensing power is made very stable. Thus, stable pulse emission is achieved with this pattern if the wavelength is constant.

FIG. 6 shows Comparative Example 2 of a pulse pattern. In this pattern, several tens pulses A at wavelength λa with emission energy Elamp_a and several tens pulses B at wavelength λb with emission energy Elamp_b are alternately repeated. The drawing shows the changes in the laser medium temperature Tlod before and after the switching from pulses A to pulses B.

As shown in FIG. 6, the laser medium temperature Tlod changes all the time. The temperature changes stepwise even during the emission of pulses at the same wavelength. With such large variation in the laser medium temperature, the thermal lensing power also changes all the time, so that thermal lensing compensation is difficult, and stable pulse emission is hard to achieve.

FIG. 7 shows an example of a pulse pattern according to the present invention. This pulse pattern is a combination of pulses A and pulses B, wherein pulse trains of pulse A and pulse B, and pulse B and pulse A, are alternately repeated such as AB, BA, AB. The average lamp energy in each pulse train is constant, so that the laser medium temperature stays substantially constant. In this pattern, the same pulse (A or B) is emitted twice in succession, except for the first pulse. Therefore, the number of times of changing wavelengths is made half of the repetition frequency, whereby the unstabilizing factor associated with wavelength change is reduced.

While FIG. 2A, FIG. 2B and FIG. 7 show examples of using laser pulses at two wavelengths, pulses may be emitted at three or more wavelengths. In that case, the number of pulse types contained in a pulse train is made equal to the number of wavelengths being used, and the emission energies Elamp are set for respective wavelengths such that the average lamp energy of pulses in a pulse train is substantially constant.

If one pulse train contains pulses at three wavelengths (pulse A, pulse B, and pulse C), the order of pulses in one pulse train may be constant, or changed every time, but preferably, they should be emitted in the order that does not require complex control of the wavelength selector. From the point of view of thermal lensing compensation, pulses in each pulse train should always be in the same order (e.g., ABC), so that the laser medium temperature will be the same throughout the pulses at the same wavelength. The number of pulse types (i.e., wavelengths) in one pulse train should preferably be two or more and not more than four because of the necessity to carry out a PAT measurement quickly.

### <Warm-up process>

Since the PAT apparatus is used for carrying out functional analysis of biological tissue based on photoacoustic signals obtained from an initial pulse train, it is essential to make the laser output stable from the start of pulse emission in order to achieve higher diagnosis accuracy. For stable laser output, a warm-up process is therefore necessary before the in-vivo irradiation, so that the laser medium temperature reaches the same steady state as it is during the actual in-vivo irradiation.

For this purpose, a shutter mechanism may be provided inside the cavity to bring the laser medium temperature to the steady state in the warm-up process without emitting light. With the shutter closed, and the temperature of the circulating water around the laser medium maintained constant, the flash lamp is turned on repeatedly in the same pattern as that of the lamp emission energy during actual in-vivo irradiation, to bring the laser medium temperature to a desired steady state.

After the warm-up process, for swift transition to actual irradiation process, it is preferable to check if the laser medium temperature is in the steady state before deciding to proceed to in-vivo irradiation with the laser light. The energy output of the laser is therefore directly measured before the in-vivo irradiation, and when the energy output has become stable, the temperature of the laser medium is determined to be in the steady state.

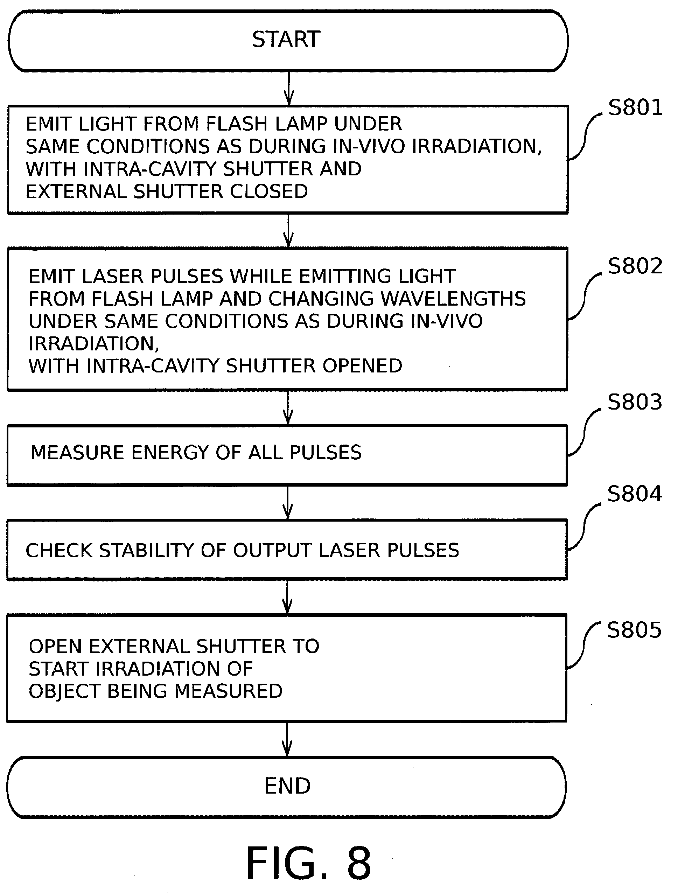

FIG. 8 shows the control procedure of laser emission according to the present invention.

Step S801 is the process of turning on the flash lamp in the same emission pattern as that when irradiating an object such as a living body, with the intra-cavity shutter and the external shutter closed. This corresponds to the warm-up process described above. There may be large changes in thermal lensing power at the start of emission from the lamp and an unstable light beam generated in the cavity may damage the optical elements inside the cavity. Thus, the warm-up process should preferably be started with the intra-cavity shutter closed to stop laser emission. The external shutter should also be closed to avoid influence on the object.

Step S802 is the process of emitting pulses while changing the wavelengths and emitting light from the flash lamp under the same conditions as when irradiating an object such as a living body, with the intra-cavity shutter opened. The same laser pulse train as that during the in-vivo irradiation is repeatedly emitted. In this process, the external shutter disposed in front of the living body is closed to shield the object from the laser light.

Step S803 is the process of measuring the energy output of all the pulses with the use of an energy meter disposed in front of the external shutter.

Step S804 is the process of checking whether the energy output of the laser determined by the energy meter has become stable at each wavelength, to decide whether or not irradiation of the object can be started. A standard deviation of energy output per a predetermined number of pulses is calculated, and the measurement is repeated until the standard deviation becomes a predetermined stabilization value or lower. In an application where a PAT measurement is carried out in accordance with this laser control method, the laser stabilization value may be set in accordance with the desired accuracy required for the PAT measurement.

Step S805 is the process of starting irradiation of the object being measured with laser light. The laser emission may be started automatically after the energy output of the laser has become stable. Alternatively, the operator may be notified of the energy output having stabilized, and start laser emission at a desired timing.

PAT measurement accuracy can be increased by the operation flow described above.

To measure the function of biological tissue with the PAT diagnosis technique, the object is irradiated on the same measurement point with laser light at different wavelengths to obtain photoacoustic signals from respective wavelengths, whereby functional information of the biological tissue can be extracted. It is preferable that the living body be kept still as much as possible to maintain its position relative to the PAT apparatus so that the photoacoustic signals from respective wavelengths can be compared precisely. With a handheld PAT apparatus, in particular, it is crucial to minimize the movement of the object. The handheld PAT apparatus has a light exit and a receiver in a probe, and the operator holds and presses this probe onto the object for the measurement. Thus there is the problem that the position of the object relative to the PAT apparatus changes all the time.

With the use of a laser pulse train consisting of pulses at two wavelengths around 800 nm repeated at a frequency of 20 Hz, for example, the measurement frequency of each wavelength is 10 Hz, which is sufficiently frequent for measuring the functional information of blood flow. Therefore, accurate measurement is possible, with reduced influence of positional displacement during the measurement. Accordingly, this method of obtaining photoacoustic signals while changing the wavelengths continuously can effectively improve the accuracy of photoacoustic spectral measurement.

### <Embodiment 1>

One configuration of a PAT apparatus in which the tunable pulsed laser of the present invention is incorporated, and a drive method thereof will be described below.

### (Configuration of apparatus)

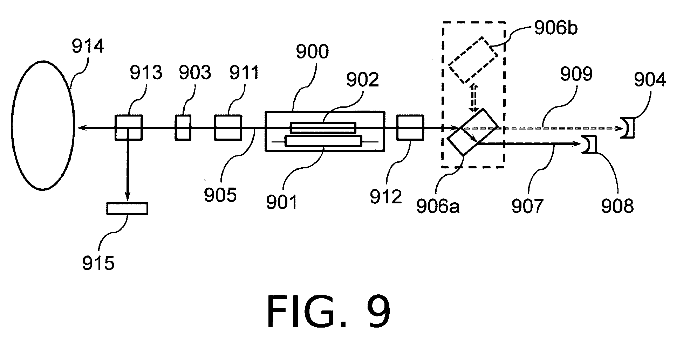

FIG. 9 is a schematic diagram of a tunable pulsed laser incorporated in a PAT apparatus. This laser apparatus is an alexandrite laser capable of emitting pulses at wavelengths of 800 nm and 755 nm with a repetition frequency of 20 Hz.

The laser apparatus is configured as follows. Inside an excitation chamber 900 are arranged a flash lamp 901 that excites alexandrite and an alexandrite crystal 902. The alexandrite crystal is immersed in circulating water at 75°C. The parallel plate 906 is made of synthetic quartz, with both surfaces optically polished to have a flatness of λ/20 and parallelism of 1 arc second or less. The plate has a thickness of 30 mm.

The output mirror 903 is coated with a dielectric film having a transmissivity of 40% to light of wavelengths of 800 nm and 755 nm. The reflective mirror 904 is coated with a reflective film of dielectric material having high reflectance to light of a center wavelength of 800 nm, and the reflective mirror 908 is coated with a reflective film of dielectric material having high reflectance to light of a center wavelength of 755 nm. When the parallel plate 906 is retracted from the optical axis 905 (position 906b), the light beam travels along the first light path 909 that coincides with the optical axis 905 and resonates. When the parallel plate 906 is inserted onto the optical axis 905 (position 906a), the light beam is moved parallel, passes the branch path 907 and resonates.

A Q-switch 911 consisting of a Pockels cell is disposed on the optical axis 905 between the output mirror 903 and the excitation chamber 900. An intra-cavity shutter 912 is disposed between the excitation chamber 900 and the parallel plate.

A handheld PAT apparatus equipped with the tunable pulsed laser described above was used to measure an object 914, which is a sample simulating a living body (phantom) in which an absorbing body simulating a blood vessel having an oxygen saturation of 80% is embedded.

### (Drive method)

The laser drive process of this embodiment will be described step by step in accordance with the flowchart of FIG. 8.

At step S801, the warm-up process is started, wherein pulses A with emission energy 60J and pulses B with emission energy 48J are emitted from the lamp, with the external shutter 913 and the intra-cavity shutter 912 closed.

After ten minutes of warming up, laser oscillation is started at step S802, with the external shutter 913 closed and the intra-cavity shutter 912 opened. With the parallel plate 906 retracted from the optical axis 905 (position 906b), the capacitor is charged for the flash lamp to emit light. After the charging, the flash lamp emits light, and the Q-switch is triggered after 150 µsec, when the energy output reaches the peak after the light emission. The light beam resonating between the output mirror 903 and the reflective mirror 904 is then emitted as pulse A, with a wavelength of 800 nm, pulse width of 50 nsec, energy output of 150 mJ/pulse, and spectral line width of 8 nm (full width at half maximum).

After emitting pulses A, the parallel plate 906 is inserted onto the optical axis 905 (position 906a) between the alexandrite crystal 902 and the reflective mirror 904. The capacitor starts to be charged to move the parallel plate 906 parallel as well as to emit light from the flash lamp. After the parallel plate 906 has been inserted, the flash lamp emits light. The Q-switch is triggered after 150 µsec after the light emission. The light beam travels along the branch path 907, which is the optical path formed when the parallel plate 906 is inserted, and resonates in the cavity formed by the output mirror 903 and the reflective mirror 908. The light beam is emitted as pulse B with a wavelength of 755 nm, pulse width of 50 ns, energy output of 150 mJ/pulse, and spectral line width of 8 nm (full width at half maximum).

The pulse train of pulse A and pulse B is repeatedly emitted.

At step S803, the energy output of respective laser pulses is measured, with an energy meter (not shown) that measures the energy of the light reflected by the external shutter.

At step S804, a standard deviation/average energy that indicates the stability of energy output is calculated per 20 pulses each of pulses A and pulses B. The energy measurement is repeated until the calculated value becomes 2.5% or lower. When the calculated value has become 2.5% or lower, the operator is notified of the fact that the energy output of the laser has been stabilized and the thermal lensing power is stable, as the laser medium temperature is now in the steady state.

At step S805, the external shutter is opened, to emit stable laser pulse train to the object 914.

### (PAT apparatus)

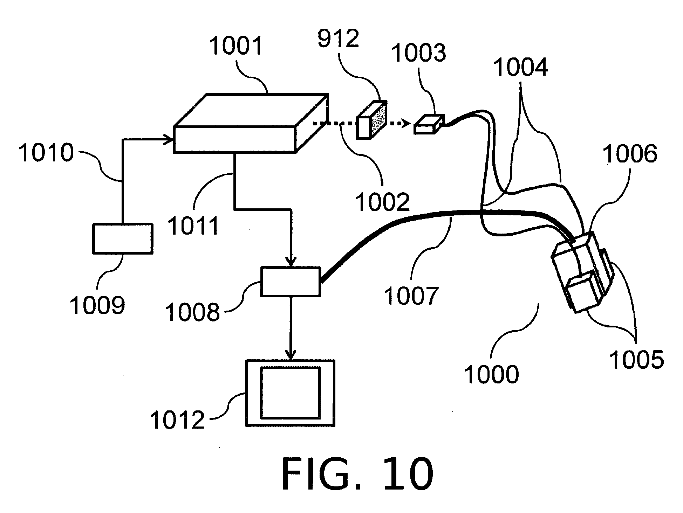

FIG. 10 shows a schematic diagram of a handheld PAT apparatus as one example of a PAT apparatus. The present invention is not limited to a handheld type and may be applied to other types, for example, in which the patient lies face down, or stands.

A laser beam 1002 exiting the tunable pulsed laser apparatus 1001 enters a fiber bundle input end 1003 when the external shutter 912 is opened. The laser beam passes through the fiber bundle 1004 and is projected onto an object (such as a phantom simulating a living body) from a fiber bundle exit end 1005 that forms a handheld probe 1000.

A photoacoustic wave generated from the object is picked up by a receiver 1006 which is a component of the handheld probe 1000 and converted into a photoacoustic signal, sent to a processor 1008 via a signal line 1007 and processed. A controller 1009 outputs control signals 1010 for changing the wavelengths of the tunable pulsed laser apparatus 1001 and the output of the flash lamp.

The processor 1008 processes signals in accordance with a trigger signal 1011 from the tunable pulsed laser apparatus 1001 based on light emission. The processor 1008 performs various processing such as analog-digital conversion, image reconstruction, and noise removal to the photoacoustic signals, and outputs image information to a monitor 1012 to display a property distribution inside the object.

In this way, in a measurement using a PAT apparatus to which the present invention is applied, pulses A and pulses B at two different wavelengths (800 nm and 755 nm) are projected stably to a phantom that simulates a living body, to obtain photoacoustic signals from a simulated blood vessel in the phantom. The concentrations of oxyhemoglobin CHbO and deoxyhemoglobin CHb may be determined by calculating the absorption coefficients at respective wavelengths from the obtained photoacoustic signals, to deduce an oxygen saturation {CHbO / (CHbO + CHb)} x 100. The oxygen saturation thus obtained by this method was 80%.

As described above, the tunable pulsed laser of the present invention is capable of stable pulse emission with reduced thermal lensing effect, and enables accurate PAT measurement with less influence of position displacement caused by movement of the operator.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

Provided is a laser apparatus including a cavity having an output mirror and a reflective mirror, a laser medium and an excitation lamp disposed inside the cavity, a wavelength selector allowing selection of a wavelength for laser light pulses to be emitted from among a plurality of wavelengths, and a controller controlling emission timing of the excitation lamp, emission energy of the excitation lamp that is determined depending on at which wavelength, of the plurality of wavelengths, the pulses are to be emitted, and emission of the pulses. The controller continuously emits a pulse train formed of a plurality of pulses including pulses of at least two wavelengths.

## Claims

1. A laser apparatus, comprising:
a cavity configured to include an output mirror (103, 903) and a reflective mirror (104, 204, 304, 307, 404, 904);
a laser medium (101, 902) disposed inside the cavity;
an excitation lamp (102, 901) for exciting the laser medium;
a wavelength selector (106, 906) configured to allow selection of at least two wavelengths for laser light pulses to be emitted from among a plurality of wavelengths; **characterized by**
a controller configured to control emission timing of the excitation lamp, emission energy of the excitation lamp that is determined depending on at which of the at least two wavelengths of the plurality of wavelengths the pulses are to be emitted, and emission of the pulses, such that the laser apparatus
repeatedly emits a pulse train formed of a plurality of pulses including pulses of the at least two wavelengths, and such that energies of the laser light pulses emitted at different wavelengths are substantially the same.

2. The laser apparatus according to claim 1, wherein
the wavelength selector selects one of a first wavelength and a second wavelength for the pulses, and
the controller is configured to control the excitation lamp such that the laser medium repeatedly emits a pulse train which is formed of two types of pulses and in which a pulse of the second wavelength is emitted successively after a pulse of the first wavelength.

3. The laser apparatus according to claim 1, wherein
the wavelength selector selects one of a first wavelength and a second wavelength for the pulses, and
the controller is configured to control the excitation lamp such that the laser medium alternately and repeatedly emits a pulse train which is formed of two types of pulses and in which a pulse of the second wavelength is emitted successively after a pulse of the first wavelength, and a pulse train which is formed two types of pulses and in which the pulse of the first wavelength is emitted successively after the pulse of the second wavelength.

4. The laser apparatus according to any one of claims 1 to 3, further comprising a shutter disposed inside the cavity, wherein
the controller performs a warm-up process in which the excitation lamp is turned on with the shutter closed so that no pulses are emitted, so as to bring the laser medium to a steady temperature state.

5. The laser apparatus according to any one of claims 1 to 4, further comprising an energy meter configured to measure energy output of the pulses, wherein
the controller is adapted to decide whether or not the laser medium is in a steady temperature state based on whether or not the energy output has stabilized.

6. The laser apparatus according to any one of claims 1 to 5, wherein
the reflective mirror has a curvature that compensates for a thermal lensing effect in the laser medium.

7. The laser apparatus according to any one of claims 1 to 5, further comprising a telescope disposed between the laser medium and the wavelength selector, wherein
the telescope compensates for a thermal lensing effect in the laser medium by adjusting lens-to-lens distance.

8. A photoacoustic apparatus, comprising:
the laser apparatus according to any one of claims 1 to 7;
a receiver configured to receive an acoustic wave generated from an object that has absorbed pulses of laser light emitted from the laser apparatus; and
a processor configured to obtain a property distribution of inside of the object based on the acoustic wave.

9. The photoacoustic apparatus according to claim 8 dependent on claim 2 or 3;
the two wavelengths respectively correspond to absorption characteristics of oxyhemoglobin and deoxyhemoglobin.

10. A control method for a laser apparatus that includes a cavity including an output mirror (103, 903) and a reflective mirror (104, 204, 304, 307, 404, 904), a laser medium (101, 902) disposed inside the cavity, an excitation lamp (102, 901) for exciting the laser medium, a wavelength selector (106, 906) allowing selection of at least two wavelengths for laser light pulses to be emitted from among a plurality of wavelengths,
**characterized in that** the apparatus comprises a controller controlling emission timing of the excitation lamp, emission energy of the excitation lamp that is determined depending on at which of the at least two wavelengths of the plurality of wavelengths the pulses are to be emitted, and emission of the pulses,
the method comprising an emission step of operating the controller such that the laser apparatus repeatedly emits a pulse train formed of a plurality of pulses including pulses of the at least two wavelengths, and such that energies of the laser light pulses emitted at different wavelengths are substantially the same.

11. The laser apparatus control method according to claim 10, further comprising a selection step of operating the wavelength selector to select one of a first wavelength and a second wavelength for the pulses, wherein
the controller is configured to control the excitation lamp such that the laser medium repeatedly emits a pulse train which is formed of two types of pulses and in which a pulse of the second wavelength is emitted successively after a pulse of the first wavelength, in the emission step.

12. The laser apparatus control method according to claim 10, further comprising a selection step of operating the wavelength selector to select one of a first wavelength and a second wavelength for the pulses, wherein
the controller is configured to control the excitation lamp such that the laser medium alternately and repeatedly emits a pulse train which is formed of two types of pulses and in which a pulse of the second wavelength is emitted successively after a pulse of the first wavelength, and a pulse train which is formed two types of pulses and in which the pulse of the first wavelength is emitted successively after the pulse of the second wavelength, in the emission step.

13. The laser apparatus control method according to any one of claims 10 to 12, wherein the laser apparatus further includes a shutter disposed inside the cavity, and
the method further comprises, before the emission step, a warm-up step of operating the controller to perform a warm-up process in which the excitation lamp is turned on with the shutter closed so that no pulses are emitted, so as to bring the laser medium to a steady temperature state.

14. The laser apparatus control method according to any one of claims 10 to 13, wherein the laser apparatus further includes an energy meter for measuring energy output of the pulses, and
the method further comprises, before the emission step, a step of operating the controller to decide whether or not the laser medium is in a steady temperature state based on whether or not the energy output has stabilized.

## Patentansprüche

1. Laservorrichtung mit:
einer Kavität, die konfiguriert ist, einen Ausgabespiegel (103, 903) und einen reflektierenden Spiegel (104, 204, 304, 307, 404, 904) zu enthalten;
einem Lasermedium (101, 902), das innerhalb der Kavität angeordnet ist;
einer Anregungslampe (102, 901) zum Anregen des Lasermediums;
einem Wellenlängenselektor (106, 906), der konfiguriert ist, eine Auswahl von zumindest zwei Wellenlängen aus einer Vielzahl von Wellenlängen für zu emittierende Laserlichtpulse zu ermöglichen; **gekennzeichnet durch**
eine Steuerungseinheit, die konfiguriert ist, eine Emissionszeit der Anregungslampe, eine Emissionsenergie der Anregungslampe, die abhängig davon bestimmt wird, welche der zumindest zwei Wellenlängen der Vielzahl von Wellenlängen der Pulse zu emittieren sind, und eine Emission der Pulse zu steuern,
sodass die Laservorrichtung wiederholt eine Pulsfolge, die aus einer Vielzahl von Pulsen einschließlich von Pulsen der zumindest zwei Wellenlängen emittiert, und
sodass Energien der Laserlichtpulse, die mit den verschiedenen Wellenlängen emittiert werden, im Wesentlichen dieselben sind.

2. Laservorrichtung nach Anspruch 1, wobei
der Wellenlängenselektor eine aus einer ersten Wellenlänge und einer zweiten Wellenlänge für die Pulse auswählt, und
die Steuerungseinheit konfiguriert ist, die Anregungslampe so zu steuern, dass das Lasermedium wiederholt eine Pulsfolge emittiert, die aus zwei Typen von Pulsen gebildet ist und bei der ein Puls der zweiten Wellenlänge sukzessive nach einem Puls der ersten Wellenlänge emittiert wird.

3. Laservorrichtung nach Anspruch 1, wobei
der Wellenlängenselektor einer aus einer ersten Wellenlänge und einer zweiten Wellenlänge für die Pulse auswählt, und
die Steuerungseinheit konfiguriert ist, die Anregungslampe so zu steuern, dass das Lasermedium alternierend und wiederholt eine Pulsfolge, die aus zwei Typen von Pulsen gebildet ist, und bei der ein Puls der zweiten Wellenlänge sukzessive nach einem Puls der ersten Wellenlänge emittiert wird, und eine Pulsfolge, die aus zwei Typen von Pulsen gebildet ist, und bei der der Puls der ersten Wellenlänge sukzessive nach dem Puls der zweiten Wellenlänge emittiert wird, emittiert.

4. Laservorrichtung nach einem der Ansprüche 1 bis 3, ferner mit einem Shutter, der innerhalb der Kavität angeordnet ist, wobei
die Steuerungseinheit einen Aufwärmprozess durchführt, in dem die Anregungslampe eingeschaltet wird, wobei der Shutter geschlossen ist, sodass keine Pulse emittiert werden, um so das Lasermedium in einen Gleichgewichtstemperaturzustand zu bringen.

5. Laservorrichtung nach einem der Ansprüche 1 bis 4, ferner mit einer Energiemesseinheit, die konfiguriert ist, eine Energieausgabe der Pulse zu messen, wobei
die Steuerungseinheit angepasst ist, basierend darauf, ob die Energieausgabe stabilisiert wurde oder nicht, zu entscheiden, ob das Lasermedium in einem Gleichgewichtstemperaturzustand ist oder nicht.

6. Laservorrichtung nach einem der Ansprüche 1 bis 5, wobei
der reflektierende Spiegel eine Krümmung hat, die einen thermischen Linseneffekt in dem Lasermedium kompensiert.

7. Laservorrichtung nach einem der Ansprüche 1 bis 5, ferner mit einem Teleskop, das zwischen dem Lasermedium und dem Wellenlängenselektor angeordnet ist, wobei
das Teleskop einen thermischen Linseneffekt in dem Lasermedium durch Anpassen eines Linse-zu-Linse-Abstands kompensiert.

8. Fotoakustische Vorrichtung mit:
der Laservorrichtung nach einem der Ansprüche 1 bis 7;
einem Empfänger, der konfiguriert ist, eine akustische Welle zu empfangen, die aus einem Objekt, das Pulse des von der Laservorrichtung emittierten Laserlichts absorbiert hat, erzeugt wurden; und
einem Prozessor, der konfiguriert ist, eine Eigenschaftsverteilung des Inneren des Objekts basierend auf der akustischen Welle zu erhalten.

9. Fotoakustische Vorrichtung nach Anspruch 8 abhängig von Anspruch 2 oder 3, wobei die zwei Wellenlängen jeweils Absorptionscharakteristiken von Oxyhämoglobin und Deoxyhämoglobin entsprechen.

10. Steuerungsverfahren für eine Laservorrichtung, die eine Kavität mit einem Ausgabespiegel (103, 903) und einem reflektierenden Spiegel (104, 204, 304, 307, 404, 904), ein Lasermedium (101, 902), das innerhalb der Kavität angeordnet ist, eine Anregungslampe (102, 901) zum Anregen des Lasermediums, einen Wellenlängenselektor (106, 906), der eine Auswahl von zumindest zwei Wellenlängen aus einer Vielzahl von Wellenlängen für zu emittierende Laserlichtpulse ermöglicht, enthält,
**dadurch gekennzeichnet, dass**
die Vorrichtung eine Steuerungseinheit aufweist, die eine Emissionszeit der Anregungslampe, eine Emissionsenergie der Anregungslampe, die abhängig davon bestimmt wird, welche der zumindest zwei Wellenlängen der Vielzahl von Wellenlängen der Pulse zu emittieren sind, und eine Emission der Pulse steuert, wobei
das Verfahren einen Emissionsschritt des Betreibens der Steuerungseinheit enthält,
sodass die Laservorrichtung wiederholt eine Pulsfolge emittiert, die aus einer Vielzahl von Pulsen einschließlich von Pulsen der zumindest zwei Wellenlängen gebildet wird, emittiert und
sodass Energien der Laserlichtpulse, die bei verschiedenen Wellenlängen emittiert werden, im Wesentlichen dieselben sind.

11. Laservorrichtungssteuerungsverfahren nach Anspruch 10, ferner mit einem Selektionsschritt des Betreibens des Wellenlängenselektors, um eine aus einer ersten Wellenlänge und einer zweiten Wellenlänge für die Pulse auszuwählen, wobei
die Steuerungseinheit so konfiguriert ist, dass sie die Anregungslampe so steuert, dass das Lasermedium wiederholt eine Pulsfolge emittiert, die aus zwei Typen von Pulsen gebildet ist, und bei der ein Puls der zweiten Wellenlänge sukzessive nach einem Puls der ersten Wellenlänge in dem Emissionsschritt emittiert wird.

12. Laservorrichtungssteuerungsverfahren nach Anspruch 10, ferner mit einem Selektionsschritt des Betreibens des Wellenlängenselektors so, dass eine aus einer ersten Wellenlänge und einer zweiten Wellenlänge für die Pulse ausgewählt wird, wobei
die Steuerungseinheit konfiguriert ist, die Anregungslampe so zu steuern, dass das Lasermedium alternierend und wiederholt eine Pulsfolge, die aus zwei Typen von Pulsen gebildet ist, und bei der ein Puls der zweiten Wellenlänge sukzessive nach einem Puls der ersten Wellenlänge emittiert wird, und eine Pulsfolge, die aus zwei Typen von Pulsen gebildet ist, und bei der der Puls der ersten Wellenlänge sukzessive nach dem Puls der zweiten Wellenlänge in dem Emissionsschritt emittiert wird, emittiert.

13. Laservorrichtungssteuerungsverfahren nach einem der Ansprüche 10 bis 12, wobei die Laservorrichtung ferner einen Shutter enthält, der innerhalb der Kavität angeordnet ist, und wobei
das Verfahren ferner vor dem Emissionsschritt einen Aufwärmschritt zum Betreiben der Steuerungseinheit aufweist, um einen Aufwärmprozess durchzuführen, bei dem die Anregungslampe eingeschaltet wird, wobei der Shutter geschlossen ist, sodass keine Pulse emittiert werden, um so das Lasermedium in einen Gleichgewichtstemperaturzustand zu bringen.

14. Laservorrichtungssteuerungsverfahren nach einem der Ansprüche 10 bis 13, wobei die Laservorrichtung ferner eine Energiemesseinheit zum Messen einer Energieausgabe der Pulse enthält, und wobei
das Verfahren ferner vor dem Emissionsschritt einen Schritt des Betreibens der Steuerungseinheit aufweist, um basierend darauf, ob die Energieausgabe stabilisiert wurde oder nicht, zu entscheiden, ob das Lasermedium in einem Gleichgewichtszustand ist.

## Revendications

1. Appareil de laser, comprenant :
une cavité configurée de façon à comprendre un miroir de sortie (103, 903) et un miroir réfléchissant (104, 204, 304, 307, 404, 904) ;
un milieu de laser (101, 902) disposé à l'intérieur de la cavité ;
une lampe d'excitation (102, 901) pour exciter le milieu de laser ;
un sélecteur de longueur d'onde (106, 906), configuré de façon à permettre la sélection d'au moins deux longueurs d'onde pour des impulsions de lumière de laser devant être émises parmi une pluralité de longueurs d'onde ; **caractérisé par** :
un dispositif de commande, configuré de façon à commander un minutage d'émission de la lampe d'excitation, une énergie d'émission de la lampe d'excitation, qui est déterminée en fonction de celles des longueurs d'onde au nombre d'au moins deux de la pluralité de longueurs d'onde auxquelles les impulsions doivent être émises, et l'émission des impulsions, de telle sorte que l'appareil de laser émette de façon répétée un train d'impulsions constitué par une pluralité d'impulsions comprenant des impulsions aux longueurs d'onde au nombre d'au moins deux, et de telle sorte que des énergies des impulsions de lumière de laser émises à des longueurs d'onde différentes soient sensiblement identiques.

2. Appareil de laser selon la revendication 1, dans lequel :
le sélecteur de longueur d'onde sélectionne l'une d'une première longueur d'onde et d'une deuxième longueur d'onde pour les impulsions, et
le dispositif de commande est configuré de façon à commander la lampe d'excitation de telle sorte que le milieu de laser émette de façon répétée un train d'impulsions qui est constitué par deux types d'impulsions, et dans lequel une impulsion à la deuxième longueur d'onde est émise en succession après une impulsion à la première longueur d'onde.

3. Appareil de laser selon la revendication 1, dans lequel :
le sélecteur de longueur d'onde sélectionne l'une d'une première longueur d'onde et d'une deuxième longueur d'onde pour les impulsions, et
le dispositif de commande est configuré de façon à commander la lampe d'excitation de telle sorte que le milieu de laser émette en alternance et de façon répétée un train d'impulsions qui est constitué par deux types d'impulsions, et dans lequel une impulsion à la deuxième longueur d'onde est émise en succession après une impulsion à la première longueur d'onde, et un train d'impulsion qui est constitué par deux types d'impulsions, et dans lequel l'impulsion à la première longueur d'onde est émise en succession après l'impulsion à la deuxième longueur d'onde.

4. Appareil de laser selon l'une quelconque des revendications 1 à 3, comprenant de plus un obturateur disposé à l'intérieur de la cavité, dans lequel :
le dispositif de commande effectue un processus d'échauffement, dans lequel la lampe d'excitation est allumée avec l'obturateur fermé de telle sorte qu'aucune impulsion ne soit émise, de façon à amener le milieu de laser à un état de température stable.

5. Appareil de laser selon l'une quelconque des revendications 1 à 4, comprenant de plus un dispositif de mesure d'énergie configuré de façon à mesurer une sortie d'énergie des impulsions, dans lequel :
le dispositif de commande est adapté de façon à décider si oui ou non le milieu de laser est dans un état de température stable en fonction du fait que oui ou non la sortie d'énergie s'est stabilisée.

6. Appareil de laser selon l'une quelconque des revendications 1 à 5, dans lequel :
le miroir réfléchissant a une courbure qui compense un effet de lentille thermique dans le milieu de laser.

7. Appareil de laser selon l'une quelconque des revendications 1 à 5, comprenant de plus un télescope disposé entre le milieu de laser et le sélecteur de longueur d'onde, dans lequel :
le télescope compense un effet de lentille thermique dans le milieu de laser par ajustement de la distance d'une lentille à l'autre.

8. Appareil photoacoustique, comprenant :
l'appareil de laser selon l'une quelconque des revendications 1 à 7 ;
un récepteur configuré de façon à recevoir une onde acoustique générée à partir d'un objet qui a absorbé des impulsions de lumière de laser émises à partir de l'appareil de laser ; et
un processeur configuré de façon à obtenir une distribution de propriétés de l'intérieur de l'objet en fonction de l'onde acoustique.

9. Appareil photoacoustique selon la revendication 8 lorsqu'elle dépend de la revendication 2 ou 3 ;
les deux longueurs d'onde correspondant respectivement à des caractéristiques d'absorption de l'oxyhémoglobine et de la désoxyhémoglobine.

10. Procédé de commande pour un appareil de laser qui comprend une cavité comprenant un miroir de sortie (103, 903) et un miroir réfléchissant (104, 204, 304, 307, 404, 904), un milieu de laser (101, 902) disposé à l'intérieur de la cavité, une lampe d'excitation (102, 901) pour exciter le milieu de laser, un sélecteur de longueur d'onde (106, 906) permettant la sélection d'au moins deux longueurs d'onde pour des impulsions de lumière de laser devant être émises parmi une pluralité de longueurs d'onde,
**caractérisé en ce que** l'appareil comprend un dispositif de commande commandant un minutage d'émission de la lampe d'excitation, une énergie d'émission de la lampe d'excitation, qui est déterminée en fonction de celles des longueurs d'onde au nombre d'au moins deux de la pluralité de longueurs d'onde auxquelles les impulsions doivent être émises, et l'émission des impulsions,
le procédé comprenant une étape d'émission, consistant à faire fonctionner le dispositif de commande de telle sorte que l'appareil de laser émette de façon répétée un train d'impulsions constitué par une pluralité d'impulsions comprenant des impulsions aux longueurs d'onde au nombre d'au moins deux, et de telle sorte que des énergies des impulsions de lumière de laser émises à des longueurs d'onde différentes soient sensiblement identiques.

11. Procédé de commande d'appareil de laser selon la revendication 10, comprenant de plus une étape de sélection consistant à faire fonctionner le sélecteur de longueur d'onde de façon à sélectionner l'une d'une première longueur d'onde et d'une deuxième longueur d'onde pour les impulsions, dans lequel :
le dispositif de commande est configuré de façon à commander la lampe d'excitation de telle sorte que le milieu de laser émette de façon répétée un train d'impulsions qui est constitué par deux types d'impulsions, et dans lequel une impulsion à la deuxième longueur d'onde est émise en succession après une impulsion à la première longueur d'onde, dans l'étape d'émission.

12. Procédé de commande d'appareil de laser selon la revendication 10, comprenant de plus une étape de sélection consistant à faire fonctionner le sélecteur de longueur d'onde de façon à sélectionner l'une d'une première longueur d'onde et d'une deuxième longueur d'onde pour les impulsions, dans lequel :
le dispositif de commande est configuré de façon à commander la lampe d'excitation de telle sorte que le milieu de laser émette en alternance et de façon répétée un train d'impulsions qui est constitué par deux types d'impulsions, et dans lequel une impulsion à la deuxième longueur d'onde est émise en succession après une impulsion à la première longueur d'onde, et un train d'impulsion qui est constitué par deux types d'impulsions, et dans lequel l'impulsion à la première longueur d'onde est émise en succession après l'impulsion à la deuxième longueur d'onde, dans l'étape d'émission.

13. Procédé de commande d'appareil de laser selon l'une quelconque des revendications 10 à 12, dans lequel l'appareil de laser comprend de plus un obturateur disposé à l'intérieur de la cavité, et
le procédé comprend de plus, avant l'étape d'émission, une étape d'échauffement consistant à faire fonctionner le dispositif de commande de façon à effectuer un processus d'échauffement, dans lequel la lampe d'excitation est allumée avec l'obturateur fermé de telle sorte qu'aucune impulsion ne soit émise, de façon à amener le milieu de laser à un état de température stable.

14. Procédé de commande d'appareil de laser selon l'une quelconque des revendications 10 à 13, dans lequel l'appareil de laser comprend de plus un dispositif de mesure d'énergie pour mesurer une sortie d'énergie des impulsions, et
le procédé comprend de plus, avant l'étape d'émission, une étape consistant à faire fonctionner le dispositif de commande de façon à décider si oui ou non le milieu de laser est dans un état de température stable en fonction du fait que oui ou non la sortie d'énergie s'est stabilisée.
